# FASCICULE DE BREVET EUROPEEN

(11) **EP 1 323 729 B1**
(45) Date de publication et mention de la délivrance du brevet: **03.11.2004**
(21) Numéro de dépôt: 03290607.5
(22) Date de dépôt: 12.03.2003
(51) Int. Cl.: C07K 5/02, C07K 5/06, C07D 209/42, C07D 225/02

(54) **Nouveau procédé de synthèse de l'acide (2S, 3aS, 7aS)-perhydroindole-2-carboxylique et de ses esters, et application à la synthèse du perindopril**
Verfahren zur Synthese von (2S,3aS,7aS)-Perhydroindol-2-carbonsäure und seiner Estern, und Verwendung in der Synthese von Perindopril
Method for synthesis of (2S,3aS,7aS)-perhydroindole-2-carboxylic acid and esters thereof derivatives; and use in the synthesis of perindopril

(43) Date de publication de la demande: 02.07.2003
(73) Titulaire: LES LABORATOIRES SERVIER, 92415 Courbevoie Cédex (FR)
(72) Inventeur: Dubuffet, Thierry, 76210 Bolbec (FR); LeCouve, Jean-Pierre, 76600 le Havre (FR)

(56) Documents cités:
- WO-A-01/58868
- WO-A-03/016336
- VINCENT M ET AL: "Stereoselective Synthesis of a New Perhydroindole Derivative of Chiral Iminodiacid, a Potent Inhibitor of Agiotensin Converting Enzyme" TETRAHEDRON, ELSEVIER SCIENCE PUBLISHERS, AMSTERDAM, NL, vol. 23, no. 16, 1982, pages 1677-1680, XP002155080 ISSN: 0040-4020
- WIPF, PETER; LI, WENJIE: "Formation of Nine-Membered Lactams by Oxidative Ring Expansion of 4-Hydroxyhydroindoles: A Biomimetic Approach toward the Tuberostemonone Ring System?" JOURNAL OF ORGANIC CHEMISTRY (1999), 64(13), 4576-4577, XP002240232 ISSN: 0022-3263

## Description

La présente invention concerne un procédé de synthèse industrielle de l'acide (2S, 3aS, 7aS)-perhydroindole-2-carboxylique et de ses esters, et leur application à la synthèse industrielle du perindopril et de ses sels pharmaceutiquement acceptables.

Plus spécifiquement, la présente invention concerne un nouveau procédé de synthèse industrielle des dérivés de formule (I) : 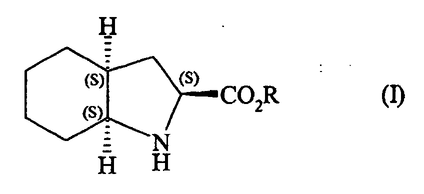 dans laquelle R représente un atome d'hydrogène ou un groupement benzyle ou alkyle (C₁-C₆) linéaire ou ramifié,
ainsi que leurs sels d'addition à un acide ou à une base minéral(e) ou organique.

Les composés de formule (I) obtenus selon le procédé de l'invention sont utiles dans la synthèse du perindopril de formule (II) : 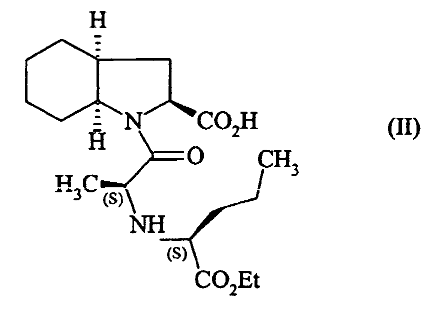 ainsi que dans celle de ses sels pharmaceutiquement acceptables.

Le perindopril, ainsi que ses sels, possèdent des propriétés pharmacologiques intéressantes.

Leur principale propriété est d'inhiber l'enzyme de conversion de l'angiotensine I (ou kininase II), ce qui permet d'une part d'empêcher la transformation du décapeptide angiotensine I en octapeptide angiotensine II (vasoconstricteur), et d'autre part de prévenir la dégradation de la bradykinine (vasodilatateur) en peptide inactif.
Ces deux actions contribuent aux effets bénéfiques du perindopril dans les maladies cardiovasculaires, tout particulièrement l'hypertension artérielle et l'insuffisance cardiaque.

Le perindopril, sa préparation et son utilisation en thérapeutique ont été décrits dans le brevet européen EP 0 049 658.

Compte-tenu de l'intérêt pharmaceutique de ce composé, il était important de pouvoir accéder à l'intermédiaire de formule (I) avec un procédé de synthèse industrielle performant, permettant l'obtention sélective du diastéréoisomère (S,S,S) avec un bon rendement et une excellente pureté, à partir de matières premières bon marché.

Quelques méthodes de préparation des composés de formule (I) sont déjà connues.

Ainsi, le brevet EP 0 037 231 utilise comme matière première l'acide indole 2-carboxylique, qui est soumis à une hydrogénation catalytique sur rhodium pour donner un mélange des deux isomères cis endo de configurations respectives (2S, 3aS, 7aS) et (2R, 3aR, 7aR). Ce mélange est ensuite séparé de façon particulièrement laborieuse : synthèse du dérivé N-benzoylé, cristallisation fractionnée du sel du diastéréoisomère avec la (S)-α-phényl-éthylamine, libération des deux dérivés (S, S, S) et (R, R, R) N-benzoylés, puis élimination du groupement benzoyle, suivie d'un passage sur colonne échangeuse d'ions et d'une recristallisation.

Le brevet EP 0 115 345, pour cette même séparation, utilise plusieurs étapes nécessitant l'estérification de la fonction acide carboxylique par l'alcool benzylique, la salification de l'amino ester par la N-benzyloxycarbonyl-(S)-phénylalanine, la séparation par cristallisation fractionnée de l'isomère (S, S, S), la libération de la fonction aminée optionnellement suivie de la libération du groupement acide carboxylique.

Les brevets EP 0 308 339 et EP 0 308 341 utilisent également comme matière première l'acide indole 2-carboxylique, qui est dans un premier temps réduit en acide indoline 2-carboxylique, pour donner un mélange d'acide indoline carboxylique 2R et 2S, lesquels sont ensuite séparés par cristallisation fractionnée. L'isomère 2S est ensuite soumis à hydrogénation catalytique pour conduire au composé de formule (I).

La demanderesse a présentement mis au point un nouveau procédé de synthèse industrielle des dérivés de formule (I), à partir d'une matière première particulièrement bon marché, et qui permet l'obtention sélective du diastéréoisomère (S, S, S) avec un bon rendement et une excellente pureté.

Plus spécifiquement, la présente invention concerne un procédé de synthèse industrielle des composés de formule (I), caractérisé en ce que l'on fait réagir la 2,7-oxepanedione de formule (III) : 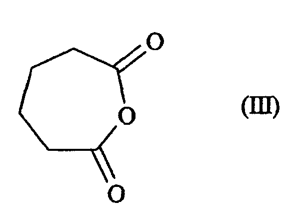 avec le composé de formule (IV) : 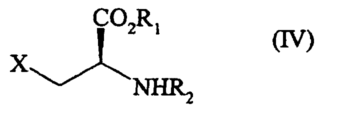 dans laquelle R₁ représente un groupement benzyle ou alkyle (C₁-C₆) linéaire ou ramifié, R₂ représente un groupement protecteur de la fonction amine qui est différent de R₁, et X représente un atome de brome ou d'iode,
en présence de zinc ou d'amalgame zinc/cuivre,
pour conduire après déprotection de la fonction amine au composé de formule (V) : 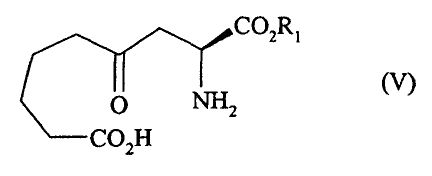 dans laquelle R₁ est tel que défini précédemment,
que l'on cyclise par réaction avec un agent de chloration tel que le chlorure de thionyle ou le chlorure d'oxalyle, ou avec un agent de couplage peptidique, pour conduire au composé de formule (VI) : 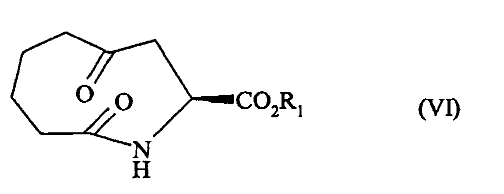 dans laquelle R₁ est tel que défini précédemment,
que l'on soumet à une réaction de couplage en présence de titane,
pour conduire au composé de formule (VII) : 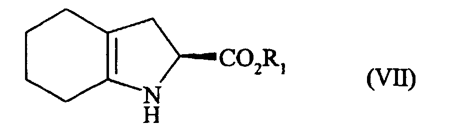 dans laquelle R₁ est tel que défini précédemment,
que l'on soumet à une hydrogénation catalytique, en présence d'un catalyseur tel que le platine, le palladium, le rhodium ou le nickel,
sous une pression comprise entre 1 et 30 bars, de préférence entre 1 et 10 bars, pour conduire, après éventuelle déprotection ou reprotection de la fonction acide, au composé de formule (I).

La présente invention concerne également une variante du procédé précédent, dans laquelle le composé intermédiaire de formule (V) est obtenu par réaction de l'acide adipique de formule (VIII) :

HO₂C-(CH₂)₄-CO₂H (VIII)

avec un agent de chloration tel que le chlorure de thionyle ou le chlorure d'oxalyle, suivie du couplage du chlorure d'acide de formule (IX) ainsi obtenu :

CIOC-(CH₂)₄-CO₂H (IX)

avec le composé de formule (IV) : 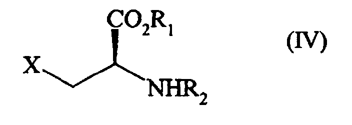 dans laquelle R₁ représente un groupement benzyle ou alkyle (C₁-C₆) linéaire ou ramifié, R₂ représente un groupement protecteur de la fonction amine qui est différent de R₁, et X représente un atome de brome ou d'iode,
en présence de zinc ou d'amalgame zinc/cuivre,
et en présence éventuelle de dichlorure de bis-(triphénylphosphine)palladium,
pour conduire après déprotection de la fonction amine au composé de formule (V).

Les composés de formule (VI) sont des produits nouveaux, utiles comme intermédiaires de synthèse dans l'industrie chimique ou pharmaceutique, notamment dans la synthèse du composé de formule (I), et font à ce titre partie intégrante de l'invention.

Le composé préféré de formule (VI) est celui pour lequel R représente le groupement benzyle.

Parmi les groupements protecteurs de la fonction amine utilisables dans le procédé de la présente invention, on peut citer à titre non limitatif les groupements tert-butyloxycarbonyle et benzyle.

Le composé de formule (I) ainsi obtenu a une très bonne pureté chimique et énantiomérique, ce qui rend son emploi particulièrement avantageux dans la synthèse du perindopril de formule (II).

A titre d'illustration, le couplage du composé de formule (I) obtenu selon le procédé de l'invention avec le composé de formule (X) : 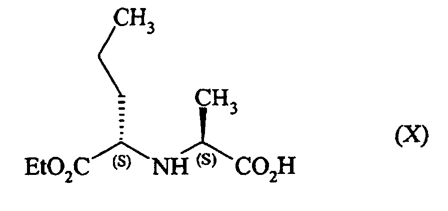 permet d'obtenir le perindopril de formule (II) avec une pureté et un rendement très satisfaisants.

Les exemples ci-dessous illustrent l'invention mais ne la limitent en aucune façon.

### EXEMPLE 1 : Acide (2S,3aS,7aS)-perhydroindole-2-carboxylique, paratoluènesulfonate

### Stade A : Acide (8S)-9-benzyloxy-8-[(tert-butyloxycarbonyl)-amino]-6,9-dioxo-nonanoïque

Dans un réacteur, placer 200 g de 2,7-oxepanedione, 1 1 de diméthylformamide, 204 g de poudre de zinc puis 758 g de (2S)-2-[(tert-butyloxycarbonyl)-amino]-3-iodopropanoate de benzyle. Laisser la température du mélange réactionnel monter sous agitation à 60°C, puis maintenir cette température pendant 1 h. Ramener ensuite le mélange réactionnel à température ambiante, le filtrer puis le verser sur une solution glacée d'acide chlorhydrique à 5 %, puis extraire par l'acétate d'éthyle et évaporer à sec.
L'acide (8S)-9-benzyloxy-8-[(tert-butyloxycarbonyl)-amino]-6,9-dioxo-nonanoïque est ainsi obtenu avec un rendement de 80 %.

### Stade B : Acide (8S)-8-amino-9-benzyloxy-6,9-dioxo-nonanoïque

Dans un réacteur, placer 200 g du composé obtenu dans le stade précédent, 1,5 l de dichlorométhane et 56 g d'acide trifluoroacétique. Après 1h30 d'agitation à température ambiante, ajouter 2 l d'une solution saturée d'hydrogénocarbonate de sodium. Extraire par le dichlorométhane et évaporer à sec.
L'acide (8S)-8-amino-9-benzyloxy-6,9-dioxo-nonanoïque est ainsi obtenu avec un rendement de 90 %.

### Stade C : (2S)-4,9-Dioxo-2-azonanecarboxylate de benzyle

Dans un réacteur, placer 200 g du composé obtenu dans le stade précédent, 2 l d'acétate d'éthyle puis 44 g de 1-hydroxybenzotriazole et 140 g de dicyclohexylcarbodiimide. Le mélange est ensuite porté à 30°C pendant 3 heures, puis il est refroidi et filtré. Le filtrat est ensuite lavé, puis évaporé à sec.
Le (2S)-4,9-dioxo-2-azonanecarboxylate de benzyle est ainsi obtenu avec un rendement de 95 %.

### Stade D: (2S)-2,3,4,5,6,7-Hexahydro-1H-indole-2-carboxylate de benzyle, paratoluènesulfonate

Dans un réacteur, placer 2 l d'une suspension de titane sur graphite 0,7 M dans le tétrahydrofurane, puis amener au reflux et ajouter lentement une solution de 200 g du composé obtenu dans le stade précédent dans 2 l de tétrahydrofurane. Amener ensuite le mélange réactionnel à température ambiante, le filtrer sur un lit de silice puis laver et évaporer le filtrat à sec. Reprendre le produit brut dans le toluène, ajouter 131 g d'acide paratoluènesulfonique monohydrate, porter la suspension au reflux et éliminer l'eau par distillation azéotropique. Refroidir le milieu à température ambiante, filtrer, laver le sel par du toluène puis le sécher.
Le paratoluènesulfonate du (2S)-2,3,4,5,6,7-hexahydro-1*H*-indole-2-carboxylate de benzyle est ainsi obtenu avec un rendement de 77 %.

### Stade E: Acide (2S, 3aS, 7aS)-perhydroindole-2-carboxylique, paratoluènesulfonate

Dans un hydrogénateur, placer 200 g du composé obtenu dans le stade précédent, en solution dans l'acide acétique, puis 5 g de Pt/C à 10 %. Hydrogéner sous pression de 5 bars à température ambiante, jusqu'à absorption de la quantité théorique d'hydrogène. Eliminer le catalyseur par filtration, puis refroidir entre 0 et 5°C et récolter le solide obtenu par filtration. Laver le gâteau et le sécher jusqu'à poids constant.
Le paratoluènesulfonate de l'acide (2S, 3aS, 7aS)-perhydroindole-2-carboxylique est ainsi obtenu avec un rendement de 87 % et une pureté énantiomérique de 99 %.

### EXEMPLE 2 : Acide (2S,3aS,7aS)-perhydroindole-2-carboxylique, paratoluènesulfonate (variante)

### Stade A : Acide (8S)-9-benzyloxy-8-[(tert-butyloxycarbonyl)-amino]-6,9-dioxononanoïque

Dans un réacteur, placer 200 g d'acide adipique, 1 1 de dichlorométhane, puis amener le mélange à 0-5°C et ajouter lentement 174 g de chlorure d'oxalyle. Agiter ensuite 2 heures à température ambiante, puis évaporer à sec.
Reprendre le résidu dans 1 l de diméthylformamide, puis ajouter 178 g d'amalgame zinc/cuivre, 1 g de dichlorure de bis-(triphénylphosphine) palladium et 666 g de (2S)-2-[(tert-butyloxycarbonyl)-amino]-3-iodopropanoate de benzyle. Laisser la température du mélange réactionnel monter sous agitation à 60°C, puis maintenir cette température pendant 1 h. Ramener ensuite le mélange réactionnel à température ambiante, le filtrer puis le verser sur une solution glacée d'acide chlorhydrique à 5 %, puis extraire par l'acétate d'éthyle et évaporer à sec.
L'acide (8S)-9-benzyloxy-8-[(tert-butyloxycarbonyl)-amino]-6,9-dioxo-nonanoïque est ainsi obtenu avec un rendement de 75 %.

### Stade B : Acide (8S)-8-amino-9-benzyloxy-6,9-dioxo-nonanoïque

Le stade B est identique au stade B de l'exemple 1.

### Stade C : (2S)-4,9-Dioxo-2-azonanecarboxylate de benzyle

Dans un réacteur, placer 200 g du composé obtenu au stade précédent, 2 l de dichlorométhane, puis amener le mélange à 0-5°C et ajouter lentement 93 g de chlorure de thionyle. Agiter ensuite 2 heures à reflux, puis, après retour à température ambiante, laver la phase organique par de l'eau puis l'évaporer à sec.
Le (2S)-4,9-dioxo-2-azonanecarboxylate de benzyle est ainsi obtenu avec un rendement de 85 %.

Les stades D et E sont identiques aux stades D et E de l'exemple 1.

### EXEMPLE 3 : (2S, 3aS, 7aS)-Perhydroindole-2-carboxylate de benzyle, paratoluènesulfonate

Dans un réacteur, chauffer à reflux 200 g du composé de l'exemple 1, 9 g d'acide paratoluènesulfonique monohydrate, 59 g d'alcool benzylique et 700 ml de toluène en éliminant l'eau formée à l'aide d'un décanteur en continu. Lorsqu'il ne décante plus d'eau, refroidir, essorer le précipité formé et sécher.
Le paratoluènesulfonate du (2S, 3aS, 7aS)-perhydroindole-2-carboxylate de benzyle est ainsi obtenu avec un rendement de 91 % et une pureté énantiomérique de 99 %.

## Revendications

1. Procédé de synthèse industrielle des composés de formule (I) : 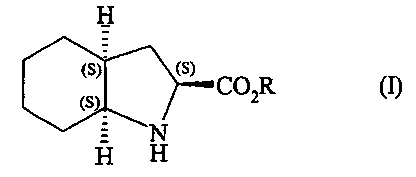 dans laquelle R représente un atome d'hydrogène ou un groupement benzyle ou alkyle (C₁-C₆) linéaire ou ramifié,
**caractérisé en ce que** l'on fait réagir la 2,7-oxepanedione de formule (III) : 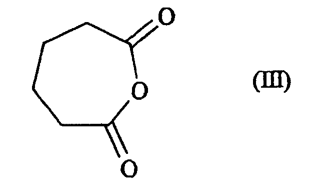 avec le composé de formule (IV) : 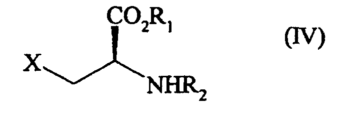 dans laquelle R₁ représente un groupement benzyle ou alkyle (C₁-C₆) linéaire ou ramifié, R₂ représente un groupement protecteur de la fonction amine qui est différent de R₁, et X représente un atome de brome ou d'iode,
en présence de zinc ou d'amalgame zinc/cuivre,
pour conduire après déprotection de la fonction amine au composé de formule (V) : 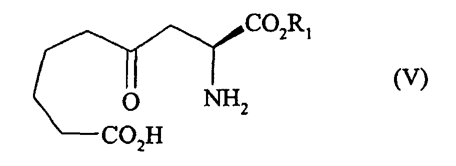 dans laquelle R₁ est tel que défini précédemment,
que l'on cyclise par réaction avec un agent de chloration tel que le chlorure de thionyle ou le chlorure d'oxalyle, ou avec un agent de couplage peptidique, pour conduire au composé de formule (VI) : 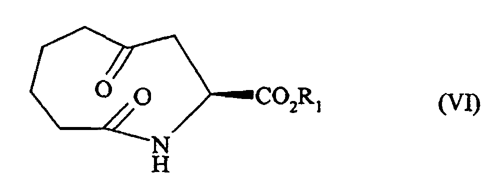 dans laquelle R₁ est tel que défini précédemment,
que l'on soumet à une réaction de couplage en présence de titane,
pour conduire au composé de formule (VII) : 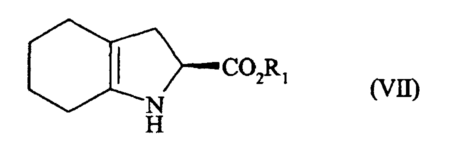 dans laquelle R₁ est tel que défini précédemment,
que l'on soumet à une hydrogénation catalytique, en présence d'un catalyseur tel que le platine, le palladium, le rhodium ou le nickel,
sous une pression comprise entre 1 et 30 bars, pour conduire, après éventuelle déprotection ou reprotection de la fonction acide, au composé de formule (I).

2. Procédé de synthèse selon la revendication 1, **caractérisé en ce que** la pression d'hydrogène est comprise entre 1 et 10 bars.

3. Procédé de synthèse selon l'une quelconque des revendications 1 ou 2, **caractérisé en ce que** le composé intermédiaire de formule (V) est obtenu par réaction de l'acide adipique de formule (VIII) :
HO₂C-(CH₂)₄-CO₂H (VIII)
avec un agent de chloration tel que le chlorure de thionyle ou le chlorure d'oxalyle, suivie du couplage du chlorure d'acide de formule (IX) ainsi obtenu :
ClOC-(CH₂)₄-CO₂H (IX)
avec le composé de formule (IV) : 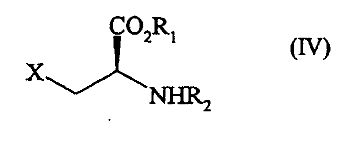 dans laquelle R₁ représente un groupement benzyle ou alkyle (C₁-C₆) linéaire ou ramifié, R₂ représente un groupement protecteur de la fonction amine qui est différent de R₁, et X représente un atome de brome ou d'iode,
en présence de zinc ou d'amalgame zinc/cuivre,
et en présence éventuelle de dichlorure de bis-(triphénylphosphine)palladium,
pour conduire après déprotection de la fonction amine au composé de formule (V).

4. Composé de formule (VI) : 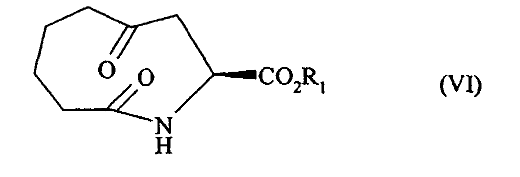 dans laquelle R₁ représente un groupement benzyle ou alkyle (C₁-C₆) linéaire ou ramifié.

5. Composé de formule (VI) selon la revendication 4, **caractérisé en ce que** R₁ représente le groupement benzyle.

6. Procédé de synthèse selon l'une quelconque des revendications 1, 2 ou 3, permettant l'obtention du dérivé de formule (I) dans laquelle R représente un atome d'hydrogène.

7. Procédé de synthèse selon l'une quelconque des revendications 1, 2 ou 3, permettant l'obtention du dérivé de formule (I) dans laquelle R représente le groupement benzyle.

8. Procédé de synthèse du perindopril ou de ses sels pharmaceutiquement acceptables à partir d'un composé de formule (I), **caractérisé en ce que** ledit composé de formule (I) est obtenu selon le procédé de la revendication 1.

## Patentansprüche

1. Verfahren zur technischen Synthese der Verbindungen der Formel (I): 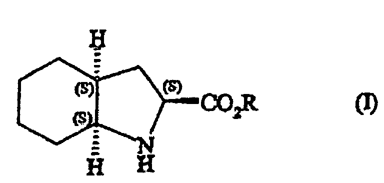 in der R ein Wasserstoffatom oder eine Benzylgruppe oder eine geradkettige oder verzweigte (C₁-C₆)-Alkylgruppe bedeutet,
**dadurch gekennzeichnet, daß** man 2,7-Oxepandion der Formel (III): 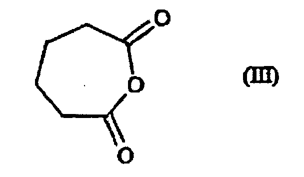 mit der Verbindung der Formel (IV): 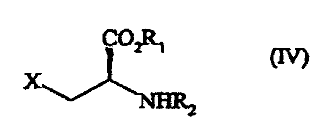 in der R₁ eine Benzylgruppe oder eine geradkettige oder verzweigte (C₁-C₆)-Alkylgruppe, R₂ eine Schutzgruppe für die Aminfunktion, die von R₁ verschieden ist, und X ein Brom- oder Iodatom bedeuten,
in Gegenwart von Zink oder Zink/Kupfer-Amalgam umsetzt,
so daß man nach der Abspaltung der Schutzgruppe der Aminfunktion die Verbindung der Formel (V) erhält: 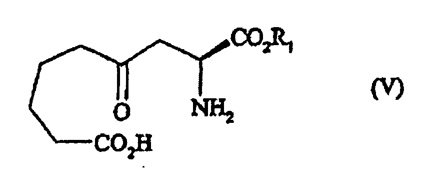 in der R₁ die oben angegebenen Bedeutungen besitzt,
welche man durch Umsetzung mit einem Chlorierungsmittel, wie Thionylchlorid oder Oxalylchlorid, oder einem Peptid-Kupplungsmittel cyclisiert zur Bildung der Verbindung der Formel (VI): 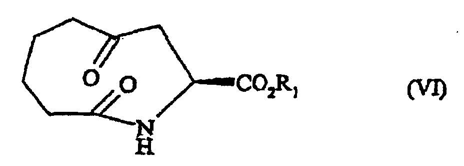 in der R₁ die oben angegebenen Bedeutungen besitzt,
welche man einer Kupplungsreaktion in Gegenwart von Titan unterwirft.
zur Bildung der Verbindung der Formel (VII): 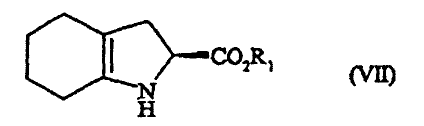 in der R₁ die oben angegebenen Bedeutungen besitzt,
welche man einer katalytischen Hydrierung in Gegenwart eines Katalysators, wie Platin, Palladium, Rhodium oder Nickel, bei einem Druck zwischen 1 und 30 bar unterwirft, so daß man nach der eventuellen Abspaltung der Schutzgruppen oder dem Schutz der Säurefunktion die Verbindung der Formel (I) erhält.

2. Syntheseverfahren nach Anspruch 1, **dadurch gekennzeichnet, daß** der Wasserstoffdruck zwischen 1 und 10 bar liegt.

3. Syntheseverfahren nach einem der Ansprüche 1 oder 2, **dadurch gekennzeichnet, daß** das Zwischenprodukt der Formel (V) erhalten worden ist durch Umsetzen von Adipinsäure der Formel (VIII):
HO₂C-(CH₂)₄-CO₂H (VIII)
mit einem Chlorierungsmittel, wie Thionylchlorid oder Oxalylchlorid,
gefolgt von einer Kupplung des in dieser Weise erhaltenen Säurechlorids der Formel (IX):
ClOC-(CH₂)₄-CO₂H (IX)
mit der Verbindung der Formel (IV): 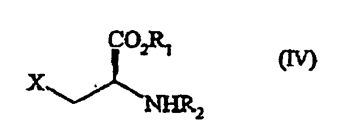 in der R₁ eine Benzylgruppe oder eine geradkettige oder verzweigte (C₁-C₆)-Alkylgruppe, R₂ eine Schutzgruppe für die Aminfunktion, die von R₁ verschieden ist, und X ein Brom- oder Iodatom bedeuten,
in Gegenwart von Zink oder Zink/Kupfer-Amalgam
und gegebenenfalls in Gegenwart von Bis(triphenylphosphin)-palladiumdichlorid, so daß man nach der Abspaltung der Schutzgruppe der Aminfunktion die Verbindung der Formel (V) erhält.

4. Verbindung der Formel (VI): 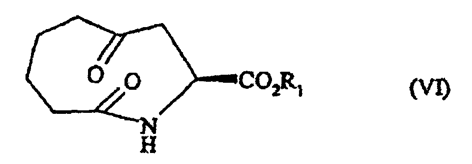 in der R₁ eine Benzylgruppe oder eine geradkettige oder verzweigte (C₁-C₆)-Alkylgruppe bedeutet.

5. Verbindung der Formel (VI) nach Anspruch 4, **dadurch gekennzeichnet, daß** R₁ die Benzylgruppe bedeutet.

6. Syntheseverfahren nach einem der Ansprüche 1, 2 oder 3 zur Herstellung des Derivats der Formel (I), in der R ein Wasserstoffatom bedeutet.

7. Syntheseverfahren nach einem der Ansprüche 1, 2 oder 3 zur Herstellung des Derivats der Formel (I), in der R die Benzylgruppe bedeutet.

8. Verfahren zur Synthese von Perindopril oder seiner pharmazeutisch annehmbaren Salze ausgehend von einer Verbindung der Formel (I), **dadurch gekennzeichnet, daß** die Verbindung der Formel (I) nach dem Verfahren von Anspruch 1 erhalten worden ist.

## Claims

1. Process for the industrial synthesis of compounds of formula (1) : 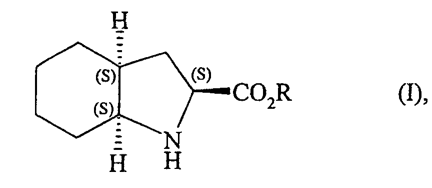 wherein R represents a hydrogen atom or a benzyl or linear or branched (C₁-C₆)alkyl group,
**characterised in that** 2,7-oxepanedione of formula (III) : 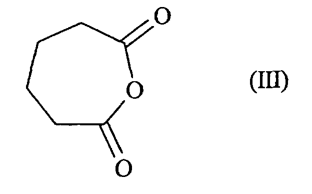 is reacted with the compound of formula (IV) : 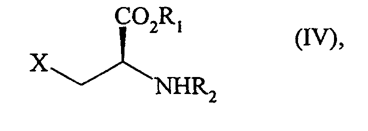 wherein R₁ represents a benzyl or linear or branched (C₁-C₆)alkyl group, R₂ represents a protecting group for the amine function which is different from R₁, and X represents a bromine or iodine atom,
in the presence of zinc or zinc/copper amalgam,
to yield, after deprotection of the amine function, the compound of formula (V) : 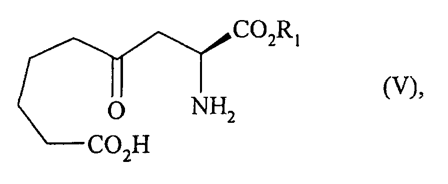 wherein R₁ is as defined hereinbefore,
which is cyclised by reaction with a chlorination agent such as thionyl chloride or oxalyl chloride or with a peptide coupling agent to yield the compound of formula (VI) : 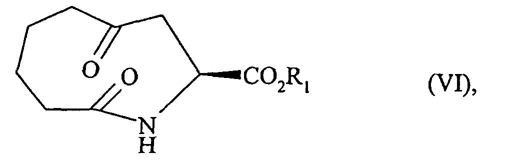 wherein R₁ is as defined hereinbefore,
which is subjected to a coupling reaction in the presence of titanium
to yield the compound of formula(VII) : 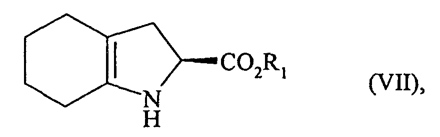 wherein R₁ is as defined hereinbefore,
which is subjected to catalytic hydrogenation, in the presence of a catalyst such as platinum, palladium, rhodium or nickel,
under a pressure of from 1 to 30 bars, to yield, after optional deprotection or reprotection of the acid function, the compound of formula (I).

2. Synthesis process according to claim 1, **characterised in that** the hydrogen pressure is from 1 to 10 bars.

3. Synthesis process according to either claim 1 or claim 2, **characterised in that** the intermediate compound of formula (V) is obtained by reaction of adipic acid of formula (VIII) :
HO₂C-(CH₂)₄-CO₂H (VIII)
with a chlorination reagent such as thionyl chloride or oxalyl chloride,
followed by coupling of the resulting acid chloride of formula (IX) :
CIOC-(CH₂)₄-CO₂H (IX)
with the compound of formula (IV) : 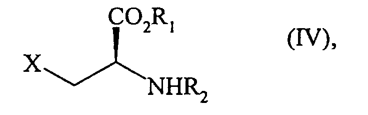 wherein R₁ represents a benzyl or linear or branched (C₁-C₆)alkyl group, R₂ represents a protecting group for the amine function which is different from R₁, and X represents a bromine or iodine atom,
in the presence of zinc or zinc/copper amalgam,
and optionally in the presence of bis(triphenylphosphine)palladium dichloride,
to yield, after deprotection of the amine function, the compound of formula (V).

4. Compound of formula (VI) : 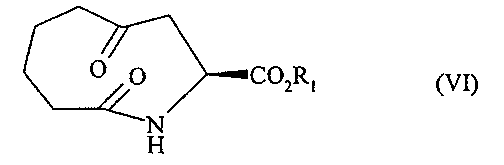 wherein R₁ represents a benzyl or linear or branched (C₁-C₆)alkyl group.

5. Compound of formula (VI) according to claim 4, **characterised in that** R₁ represents a benzyl group.

6. Synthesis process according to any one of claims 1, 2 and 3, allowing the compound of formula (I), wherein R represents a hydrogen atom, to be obtained.

7. Synthesis process according to any one of claims 1, 2 and 3, allowing the compound of formula (I), wherein R represents a benzyl group, to be obtained.

8. Process for the synthesis of perindopril or pharmaceutically acceptable salts thereof starting from a compound of formula (I), **characterised in that** the said compound of formula (I) is obtained according to the process of claim 1.
